# EUROPÄISCHE PATENTSCHRIFT

(11) **EP 0 886 778 B1**
(45) Veröffentlichungstag und Bekanntmachung des Hinweises auf die Patenterteilung: **20.06.2001**
(21) Anmeldenummer: 97902222.5
(22) Anmeldetag: 24.01.1997
(51) Int. Cl.: G01N 33/52, C12Q 1/56

(54) **SYNTHETISCHE TESTANSCHMUTZUNG**
SYNTHETIC TEST SOIL
TACHE D'ESSAI SYNTHETIQUE

(30) Priorität: 25.01.1996 DE 19602673
(43) Veröffentlichungstag der Anmeldung: 30.12.1998
(73) Patentinhaber: Pereg GmbH, 84478 Waldkraiburg (DE)
(72) Erfinder: PFEIFER, Martin, D-84478 Waldkraiburg (DE)
(74) Vertreter: Graf von Stosch, Andreas, Dr. rer. nat.
(86) Internationale Anmeldenummer: EP9700337
(87) Internationale Veröffentlichungsnummer: WO9727482

(56) Entgegenhaltungen:
- EP-A- 0 480 843
- CLINICAL CHEMISTRY, Bd. 34, Nr. 2, 1988, Seiten 430-431, XP002009810 E.M. TZVETANOVA ET AL.: "Improved Biuret method for determination of fibrinogen."

## Beschreibung

Die Erfindung betrifft eine synthetische Testanschmutzung, die Verwendung eines Fibrinklebers zur Herstellung einer solchen Anschmutzung, einen Kit zur Herstellung einer synthetischen Testanschmutzung, ein Verfahren zum Überprüfen der Wirksamkeit eines Reinigungsverfahrens, sowie ein Kit zur Durchführung dieses Verfahrens.

-Viele medizinische und chirurgische Instrumente und Apparate müssen nach ihrer Verwendung gereinigt, desinfiziert und sterilisiert werden. Reinigen und desinfizieren geschieht üblicherweise in sogenannten Waschdesinfektionsautomaten. In diesen Automaten werden die Instrumente als Vorbereitung auf die anschließende Sterilisation gereinigt, desinfiziert und getrocknet.

Das Deutsche Medizinproduktegesetz und vergleichbare Bestimmungen in anderen Ländern verlangen einen Überprüfung der Wirksamkeit des angewendeten maschinellen Reinigungsverfahrens. Dies umfaßt zum einen eine Typprüfung des Verfahrens, die der Hersteller des Waschdesinfektionsautomaten vor der Markteinführung vornehmen muß. Zum anderen muß der Anwender in regelmäßigen Abständen die Reinigungsleistung der Maschine überprüfen.

Für die Überprüfung der Reinigungsleistung sind sogenannte Prüfkörper mit definierten Testanschmutzungen erforderlich. Die hartnäckigste und am schwierigsten zu beseitigende Verschmutzung medizinischer und chirurgischer Instrumente und Apparate ist in der Regel geronnenes Blut.

Im Stand der Technik wird die vom Hersteller vorzunehmende Typprüfung daher häufig mit frischem Humanblut als Testanschmutzung durchgeführt. Frisches Blut ist erforderlich, da bei gelagertem Blut die Gerinnung durch Zusatz von Gerinnungshemmern beeinträchtigt ist. Der Einsatz von frischem Humanblut ist jedoch für die regelmäßige Überprüfung der Reinigungsleistung beim Anwender nicht praktikabel.

Der Stand der Technik (Fette und Seifen, 54. Jahrg., Nr. 2, 1952, S. 143-147) schlägt daher verschiedene Testanschmutzungen wie Grießbrei, Eigelb, sowie Stärke oder mehlhaltige Anschmutzungen vor. Da sich die Eigenschaften all dieser Testanschmutzungen deutlich von den Eigenschaften des Bluts unterscheiden, ist so keine oder allenfalls eine ungenaue Aussage über die Wirksamkeit des Reinigungsverfahrens gegenüber Blutanschmutzungen möglich.

Der Erfindung liegt die Aufgabe zugrunde, eine synthetische Testanschmutzung zu schaffen, die einfach handhabbar ist und hinsichtlich der Entfernbarkeit in einem Reinigungsverfahren dem Verhalten von frischem Humanblut ähnlicher ist als die Testanschmutzungen des Standes der Technik.

Die Erfindung löst diese Aufgabe dadurch, daß die synthetische Testanschmutzung Fibrin und/oder eine Fibrinvorstufe sowie Hämoglobin enthält. Im Rahmen der Erfindung soll der Begriff "Fibrin" sowohl monomeres als auch polymeres und/oder vernetztes Fibrin umfassen.

Der Begriff "synthetische Testanschmutzung" umfaßt im Rahmen der Erfindung jegliche Testanschmutzung, bei der Fibrin und/oder die Fibrinvorstufe separat (bspw. in isolierter Form, nicht als Bestandteil von menschlichem oder tierischem Nativblut) zugesetzt wurde. Der Begriff umfaßt somit nicht Testanschmutzungen, bei denen frisches oder geronnenes Nativblut mit anderen Bestandteilen vermischt wird, die jeweils kein Fibrin und keine Fibrinvorstufe enthalten.

Die Erfindung hat erkannt, daß die schwierige Entfernbarkeit einer Frischblutanschmutzung in erster Linie durch die Blutgerinnung (Umwandlung von Fibrinogen zu Fibrin und ggf. anschließende Vernetzung) bedingt ist und daß eine fibrin- bzw. fibrinvorstufenhaltige Testanschmutzung sehr ähnliche Eigenschaften im Hinblick auf die Entfernbarkeit aufweist. Dies ist insofern überraschend, als daß die Gerinnungsfaktoren nur einen sehr geringen Anteil der Blutinhaltsstoffe ausmachen. Dies sei beispielhaft erläutert. In einem Liter Humanblut sind etwa 520 ml Blutplasma enthalten, dieses Plasma enthält etwa 35 g Plasmaproteine, darin wiederum ist nur ein sehr kleiner Anteil Fibrinogen als Vorstufe des Fibrins enthalten. Die überraschende Erkenntnis der Erfindung ist nun, daß sich das Verhalten von Blut in einem Reinigungsverfahren gut durch eine synthetische Testanschmutzung annähern läßt, die Fibrin und/oder eine Fibrinvorstufe enthält, obwohl Fibrinogen/Fibrin nur einen Anteil von weniger als 0,5% der Blutinhaltsstoffe ausmacht.

Die Fibrinvorstufe ist vorzugsweise Fibrinogen. Die erfindungsgemäße Testanschmutzung kann zusätzliche Blutplasmaproteine wie bspw. Albumin enthalten, um die Bluteigenschaften noch besser anzunähern. Gegenstand der Erfindung ist somit eine synthetische Testanschmutzung, die sich reinigungstechnisch in guter Näherung wie Nativblut verhält, jedoch nicht aus Nativblut selbst besteht, sondern synthetisch aus verschiedenen Blutbestandteilen hergestellt wird, die die Anschmutzungseigenschaften von Blut wesentlich bestimmen.

Gegenstand der Erfindung ist ferner die Verwendung eines Fibrinklebers zur Herstellung einer erfindungsgemäßen syntheti schen Testanschmutzung. Handelsübliche Fibrinkleber sind Zwei- oder Mehrkomponentensysteme, eine Komponente enthält Fibrinogen als Fibrinvorstufe, die andere Komponente enthält Thrombin. Thrombin ist ein proteolytisches Enzym und einer der Blutgerinnungsfaktoren, es bewirkt die Umwandlung von Fibrinogen in Fibrin. In der Regel enthält ein Fibrinkleber auch noch den Blutgerinnungsfaktor XIII, der die Vernetzung von Fibrin zu Fibrinpolymeren initiiert. Andere übliche Bestandteile sind Ca²⁺-Ionen (Blutgerinnungsfaktor IV) als Aktivatoren der bei der Blutgerinnung wirkenden enzymatischen Systeme sowie Fibrinolytika (bspw. Plasmin oder dessen Vorstufe Plasminogen) und/oder Antifibrinolytika (bspw. Aprotinin).

Ein im Rahmen der Erfindung verwendbarer Fibrinkleberkit ist bspw. TISSUCOL® der Firma Immuno GmbH, 69126 Heidelberg.

Ein weiterer Gegenstand der Erfindung ist ein Kit zur Herstellung einer synthetischen Testanschmutzung, das folgende Komponenten aufweist:
a) eine Fibrinvorstufe
b) einen Umwandlungsinitiator für die Fibrinvorstufe,
wobei wenigstens eine dieser Komponenten zusätzlich Hämoglo-. bin enthält.

Der Begriff "Umwandlungsinitiator" umfaßt jegliche Stoffe, die die Umwandlung der Fibrinvorstufe in monomeres Fibrin und/oder die Polymerisation bzw. Vernetzung von Fibrin initiieren und/oder fördern.

Es sei darauf hingewiesen, daß die Aufzählung dieser Komponenten nicht abschließend sein muß, der Kit kann auch mehr als zwei Komponenten enthalten. So können bspw. die Fibrinvorstufe und der Umwandlungsinitiator als Feststoffe in lyophilisierter Form vorliegen, die erst mit geeigneten Lösungsmitteln in Lösung gebracht werden müssen. Diese Lösungsmittel können dann ebenfalls Bestandteil des Kits sein.

Die Komponente a) enthält vorzugsweise Fibrinogen, die Komponente b) vorzugsweise Thrombin. Komponente a) kann zusätzlich sonstige Blutplasmaproteine wie bspw. Albumin enthalten.

Häufig ist es zweckmäßig, wenn Komponente a) den Blutgerinnungsfaktor XIII enthält, der auf Fibrin vernetzend einwirkt und so die Gerinnung verstärkt. Auf diese Weise entsteht eine hartnäckige und besonders schwierig zu entfernende Testanschmutzung. Bevorzugt ist auch, daß eine Komponente des Kits Ca²⁺-Ionen (Blutgerinnungsfaktor IV) enthält. Schließlich können ggf. andere Stoffe, bspw. Fibrinolytika wie Plasminogen oder Antifibrinolytika wie Aprotinin enthalten sein.

Die Komponenten des Kits können bspw. in isotonischer Kochsalzlösung gelöst vorliegen. Alternativ können sie, wie oben schon ausgeführt, als Feststoffe (lyophilisiert) vorliegen, geeignete Lösungsmittel sind dann ebenfalls Bestandteil des Kits.

Ein weiterer Aspekt der Erfindung ist ein Verfahren zum Überprüfen der Wirksamkeit eines Reinigungsverfahrens. Es weist folgende Schritte auf:
a) Aufbringen einer synthetischen Testanschmutzung, die Fibrin und/oder eine Fibrinvorstufe enthält, auf einen Prüfkörper,
b) Unterziehen des Prüfkörpers dem zu prüfenden Reinigungsverfahren,
c) Nachweis von Resten der Testanschmutzung auf dem Prüfkörper.

Die Prüfkörper sollen im Hinblick auf Material und Oberflächengestaltung möglichst weitgehend den in der Praxis zu reinigenden Instrumenten und Apparaten gleichen. Zur Simulation der Reinigung chirurgischer Instrumente können bspw. 1 bis 2 mm dicke Edelstahlbleche mit einer Abmessung von 100 x 20 mm verwendet werden. Verschiedene Oberflächenkonturen, wie Riffelungen, Prägungen etc., können vorgesehen sein, um entsprechende "Schmutzecken" chirurgischer Instrumente zu simulieren. Die Oberfläche der Prüfkörper kann angerauht sein (bspw. mit Schleifkorn 180 (geschliffene Oberfläche nach DIN 17440 IV, Korn 180)), um die Haftung der Testanschmutzung zu verbessern und so sicherzustellen, daß der Prüfkörper eher schwerer zu reinigen ist als in der Praxis verwendete Instrumente und Apparate. Denkbar ist auch der Einsatz von Edelstahlschrauben als Prüfkörper, da Schraubenkopf und Gewinde verhältnismäßig schwierig zu reinigende Oberflächenbereiche aufweisen.

Zwecks Überprüfung der Reinigungswirkung bei Endoskopen können Prüfkörper mit innenliegenden Hohlräumen oder englumige Kunststoff- oder Gummischläuche Verwendung finden. Zumindest ein Teil der Innenräume bzw. Schlauchlumina sollte von außen sichtbar sein (bspw. durch Verwendung transparenten Schlauchmaterials), um den vorzugsweise eine optische Überprüfung umfassenden Nachweis von Resten der Testanschmutzung auf dieser Oberfläche zu ermöglichen. Das Aufbringen der synthetischen Testanschmutzung kann bspw. durch Aufsprühen der Komponenten eines Kits gemäß einem der Ansprüche 6 bis 11 oder durch sonstiges Auftragen geschehen. An den Schritt des Aufbringens kann sich vorzugsweise ein Schritt anschließen, in dem die Testanschmutzung gerinnen gelassen und/oder getrocknet wird. Das Gerinnen kann auch während des Trocknens erfolgen, so daß dann ein separater Gerinnungsschritt entbehrlich ist. Häufig wird es jedoch vorzuziehen sein, der Testanschmutzung vor dem Trocknen Zeit zum Gerinnen zu geben. Der Prüfkörper kann zu diesem Zweck bspw. ca. 10 min bei 20°C einer Umgebung mit einer relativen Luftfeuchtigkeit von 90% ausgesetzt werden. Der Begriff "Gerinnen" im Sinne der Erfindung umfaßt die Umwandlung etwaig vorhandener Fibrinvorstufen (Fibrinogen) in Fibrin. Vorzugsweise umfaßt die Gerinnung zusätzlich noch die Polymerisation der Fibrinmonomere zu einem Fibrinnetzwerk, bevorzugt unter der Einwirkung des Blutgerinnungsfaktors XIII sowie Ca²⁺ als Cofaktor (Blutgerinnungsfaktor IV). Ein solches polymerisiertes und quervernetztes Fibrinnetzwerk simuliert besonders gut diejenigen Verhältnisse, die bei einer geronnenen Blutverschmutzung medizinischer Instrumente vorhanden sind.

Das Trocken der Testanschmutzung kann bei Raumtemperatur erfolgen, geeignete Zeiträume können bspw. zwischen 1 und 24 h liegen. Auch ein Trocknen bei erhöhter Temperatur (bspw. 40°C) für bspw. 1 h ist möglich. Eine besonders hartnäckige Testanschmutzung kann geschaffen werden, wenn die ggf. geronnene und getrocknete erfindungsgemäße Testanschmutzung zusätzlich mit Desinfektionsmitteln behandelt und damit teilweise denaturiert wird. Denaturierte Blutrückstände sind besonders schwierig zu entfernen und treten in der Praxis dann auf, wenn bspw. chirurgische Instrumente entweder unmittelbar nach ihrer Benutzung oder einige Zeit später in Behälter mit Desinfektionslösung aufbewahrt werden.

Diese Vorbereitung des Prüfkörpers kann entweder beim Anwender erfolgen, alternativ können jedoch, wie weiter unten noch erläutert, Testkits geschaffen werden, die entsprechend vorbereitete Prüfkörper enthalten.

Der oder die "testverschmutzten" Prüfkörper werden anschließend dem zu prüfenden Reinigungsverfahren unterzogen. Nach Abschluß der Reinigung erfolgt ein Nachweis etwaiger Reste der Testanschmutzung auf dem Prüfkörper.

Bevorzugt und für den Anwender am einfachsten durchzuführen ist ein visueller Nachweis etwaiger Reste, bevorzugt nach Durchführung einer Farbreaktion, die auf dem Prüfkörper vorhandene Proteinreste anfärbt. Eine übliche und dem Fachmann geläufige Nachweisreaktion für Proteine ist bspw. die Biuret-Reaktion. Die folgende, nicht abschließende Aufzählung enthält weitere Beispiele für mögliche Nachweisreaktionen:

Kjeldahlsche Reaktion, Lowrysche Reaktion, Millonsche Reaktion, Ninhydrinreaktion, Paulysche Reaktion, Xanthoproteinreaktion.

Eine separate Proteinfarbreaktion kann entbehrlich sein, wenn die erfindungsgemäße Testanschmutzung Hämoglobin enthält, das ein optisches Erkennen der Restanschmutzung wesentlich erleichtert.

Eine etwas aufwendigere Möglichkeit ist die Hydrolyse der noch am Prüfkörper haftenden Proteine und eine Analyse der als Hydrolyseprodukte resultierenden Aminosäuren.

Sofern ein quantitativer Nachweis erforderlich ist, enthalten die Proteine der Testanschmutzung bevorzugt radioaktive Tracer wie bspw. ^{99m}Tc. Die Reinigungswirkung läßt sich dann durch Messung der von dem Prüfkörper ausgehenden γ-Strahlung des ^{99m}Tc vor und nach der Reinigung messen. Auf diese Weise läßt sich die erfindungsgemäße synthetische Testanschmutzung bzw. das erfindungsgemäße Prüfverfahren auch eichen, da Vergleichsmessungen mit Nativblut durchgeführt werden können, das ebenfalls mit ^{99m}Tc versehen ist. Man kann die Zusammensetzung der erfindungsgemäßen Testanschmutzung variieren und so ihr Verhalten gegenüber einer Reinigung weitestgehend dem Verhalten von Nativblut angleichen.

Gegenstand der Erfindung ist ferner ein Kit zur Durchführung des erfindungsgemäßen Verfahrens, das folgende Bestandteile enthält:
- einen Prüfkörper, der mit einer synthetischen Testanschmutzung, die Fibrin und/oder eine Fibrinvorstufe ent-. hält, versehen ist,
- Nachweisreagenzien zum Nachweis von Resten der Testanschmutzung.

Dieser Kit ermöglicht jedem Anwender eine einfache laufende Überprüfung der Wirksamkeit seines Waschdesinfektionsautomaten. Der oder die fertig vorbereiteten Prüfkörper wird/werden dem Kit entnommen und dem zu prüfenden Reinigungsverfahren unterzogen. Anschließend erfolgt mit den im Kit enthaltenden Nachweisreagenzien in der oben geschilderten Weise ein Nachweis und eine visuelle Beurteilung von Resten der Testanschmutzung.

Als Nachweisreaktion wird bevorzugt die Biuret-Reaktion verwendet. Die Nachweisreagenzien enthalten dann zum einen eine alkalische wäßrige Lösung und zum anderen eine wäßrige Lösung von Cu²⁺-Ionen. Die alkalische wäßrige Lösung enthält vorzugsweise zusätzlich einen Komplexbildner für Cu²⁺-Ionen, um ein Ausfallen von Kupfer(II)hydroxid in dem alkalischen Milieu zu vermeiden. Als geeigneter Komplexbildner kann bspw. Nitrilotriessigsäure (NTA) oder deren Salze Verwendung finden. Andere bekannte und dem Fachmann geläufige Komplexbildner (EDTA etc.) für Cu²⁺-Ionen sind ebenfalls geeignet.

Nachfolgend werden Ausführungsbeispiele der Erfindung beschrieben. Alle Prozentangaben sind Gewichtsprozent.

### Beispiel 1

In diesem Beispiel wird die Herstellung von Komponenten eines Kits zur Herstellung einer erfindungsgemäßen synthetischen Testanschmutzung beschrieben.

Komponente a):
0,4 g Fibrinogen und 200 E Blutgerinnungsfaktor XIII werden in 50 ml isotonischer (0,9%iger) Kochsalzlösung gelöst. Eine Einheit E Blutgerinnungsfaktor XIII entspricht derjenigen Aktivität, die in 1 ml frischem Normalplasma enthalten ist.

Komponente b):
2.500 I.E. Thrombin (human), 8 g Albumin und 30 mg CaCl₂•2H₂O werden in 50 ml isotonischer Kochsalzlösung gelöst. Eine Internationale Einheit (I.E.) Thrombin ist definiert als jene Aktivität, die in 0,0853 mg des 1. internationalen Standards von humanen Thrombin enthalten ist.

Bezogen auf die Gesamtflüssigkeitsmenge der beiden Komponenten (100 ml) beträgt der Fibrinogenanteil 0,4 Gew.-% und der Albuminanteil 8 Gew.-%. Diese Anteile können variiert werden (bspw. im Bereich 0,1 bis 0,5 Gew.-% bzw. 5 bis 10 Gew.-%), um die "Hartnäckigkeit" der aus den Komponenten herzustellenden Testanschmutzung zu variieren.

Die Inhaltsstoffe der Komponenten a) und b) sind als Bestandteile eines Fibrinkleberkits erhältlich, bspw. des oben schon genannten Kits TISSUCOL® der Immuno GmbH.

Der gemäß diesem Beispiel hergestellte Kit enthält den Blutgerinnungsfaktor XIII sowie Calciumchlorid als Cofaktor, um die Vernetzung von Fibrin zu einem polymeren Netzwerk zu fördern und so eine hartnäckige Verschmutzung herzustellen. Sofern eine besonders hartnäckige Testanschmutzung erwünscht ist, können die Komponenten a) und b) auch höher konzentriert werden, bspw. können die genannten Protein- und Hilfsstoffmengen in jeweils lediglich 5 ml isotonischer Kochsalzlösung gelöst werden.

### Beispiel 2

In diesem Beispiel wird die Herstellung von Komponenten eines zweiten Kits zur Herstellung einer erfindungsgemäßen synthetischen Testanschmutzung beschrieben.

Komponente a):
0,4 g Fibrinogen werden in 50 ml isotonischer (0,9%iger) Kochsalzlösung gelöst.

Komponente b):
25 I.E. Thrombin (human), 8 g Albumin, 8 g Hämoglobin und 30 mg CaCl₂•2H₂O werden in 50 ml isotonischer Kochsalzlösung gelöst.

Bezogen auf die Gesamtflüssigkeitsmenge der beiden Komponenten (100 ml) beträgt der Fibrinogenanteil 0,4 Gew.-% und der Albumin- und Hämoglobinanteil 8 Gew.-%. Diese Anteile können variiert werden (bspw. im Bereich 0,1 bis 0,5 Gew.-% bzw. 5 bis 10 Gew.-%), um die "Hartnäckigkeit" der aus den Komponenten herzustellenden Testanschmutzung zu variieren.

Bei diesem Kit ist der Thrombinanteil in Komponente b) deutlich vermindert worden, um ein Gerinnen schon während des Auftragens der Testanschmutzung zu vermeiden. Durch den Hämoglobinanteil kann eine visuelle Beurteilung etwaiger Reste der Testanschmutzung erfolgen, ohne daß eine separate Farbreaktion zur Sichtbarmachung durchgeführt werden muß.

### Beispiel 3

### Herstellung der Komponenten eines Nachweisreagenz.

Lösung 1: 3 g NaOH und 4 g NTA (Nitrilotriessigsäure) werden in 50 ml Wasser gelöst.

Lösung 2: 5 g CuSO₄•5H₂O werden in 50 ml Wasser gelöst.

Diese beiden Lösungen zusammen ergeben das Nachweisreagenz für die Biuret-Reaktion.

### Beispiel 4

### Versehen eines Prüfkörpers mit der Testanschmutzung

Als Prüfkörper wird ein Edelstahlblech der Größe 100 x 20 mm, Stärke 2 mm, verwendet. Die Oberfläche des Blechs ist eine geschliffene Oberfläche gemäß DIN 17440 IV, Korn 180.

Die Oberfläche des Prüfkörpers wird mit gleichen Volumenanteilen der Komponenten a) und b) aus Beispiel 1 benetzt. Bspw. können die beiden Komponenten a) und b) gleichzeitig auf die zu benetzende Prüfkörperoberfläche aufgesprüht oder auf andere Weise aufgetragen werden.

Anschließend läßt man die Testanschmutzung bei Raumtemperatur (20°C) und 90% relativer Luftfeuchtigkeit 10 min lang gerinnen. Das Gerinnen umfaßt die Umwandlung von Fibrinogen in Fibrin sowie die Vernetzung der Fibrinmonomere zu einem polymeren Netzwerk. Nach dem Gerinnen wird der Prüfkörper im Trokkenschrank bei 40°C 1 h lang getrocknet. Nach dem Trocknen ist der Prüfkörper einsatzfertig. Er kann, ggf. zusammen mit einem Nachweisreagenz gemäß Beispiel 3, an einen Anwender versandt werden.

### Beispiel 5

### Durchführung des erfindungsgemäßen Verfahrens

Der Prüfkörper gemäß Beispiel 4 wird in den zu prüfenden Waschdesinfektionsautomaten eingestellt und einem üblichen Reinigungszyklus unterzogen.

Gleiche Mengen der Lösungen 1 und 2 aus Beispiel 3 werden miteinander zu einem Nachweisreagenz vermischt, in das der gereinigte Prüfkörper eingetaucht wird. Die Verweildauer des Prüfkörpers im Nachweisreagenz beträgt vorzugsweise mindestens 60 s. Sofern am Prüfkörper noch Proteinreste vorhanden sind, komplexieren die Peptidbindungen und (sofern vorhanden) Tyrosinreste die Cu²⁺-Ionen zu einem purpurfarbenen Komplex. Somit sind Reste der Testanschmutzung auf der Prüfkörperoberfläche durch eine purpurfarbene Verfärbung erkennbar. Mit diesem Nachweisverfahren können Proteinreste bis hinab zu einer Grenze von etwa 1 bis 4 *µ*g/cm² auf der Metalloberfläche noch visuell erkannt werden.

## Patentansprüche

1. Synthetische Testanschmutzung, dadurch gekennzeichnet, daß sie enthält:
a) Fibrin und/oder eine Fibrinvorstufe in Verbindung mit einem Umwandlungsinitiator
b) Hämoglobin.

2. Synthetische Testanschmutzung nach Anspruch 1, dadurch gekennzeichnet, daß die Fibrinvorstufe Fibrinogen ist.

3. Synthetische Testanschmutzung nach Anspruch 1 oder 2, dadurch gekennzeichnet, daß sie zusätzliche Blutplasmaproteine enthält.

4. Synthetische Testanschmutzung nach Anspruch 3, dadurch gekennzeichnet, daß sie Albumin enthält.

5. Verwendung eines Fibrinklebers zur Herstellung einer synthetischen Testanschmutzung.

6. Kit zur Herstellung einer synthetischen Testanschmutzung, gekennzeichnet durch folgende Komponenten:
a) eine Fibrinvorstufe,
b) einen Umwandlungsinitiator für die Fibrinvorstufe,
wobei wenigstens eine dieser Komponenten zusätzlich Hämoglobin enthält.

7. Kit nach Anspruch 6, dadurch gekennzeichnet, daß Komponente a) Fibrinogen enthält.

8. Kit nach Anspruch 6 oder 7, dadurch gekennzeichnet, daß Komponente b) Thrombin enthält.

9. Kit nach einem der Ansprüche 6 bis 8, dadurch gekennzeichnet, daß Komponente a) zusätzlich Blutplasmaproteine enthält.

10. Kit nach Anspruch 9, dadurch gekennzeichnet, daß Komponente a) Albumin enthält.

11. Kit nach einem der Ansprüche 6 bis 10, dadurch gekennzeichnet, daß die Komponenten des Kits in isotonischer Kochsalzlösung gelöst vorliegen.

12. Verfahren zum Überprüfen der Wirksamkeit eines Reinigungsverfahrens, gekennzeichnet durch folgende Schritte:
a) Aufbringen einer synthetischen Testanschmutzung, die Fibrin und/oder eine Fibrinvorstufe in Verbindung mit einem Umwandlungsinitiator enthält, auf einen Prüfkörper,
b) Unterziehen des Prüfkörpers dem zu prüfenden Reinigungsverfahren,
c) Nachweis von Resten der Testanschmutzung auf dem Prüfkörper.

13. Verfahren nach Anspruch 12, dadurch gekennzeichnet, daß die Testanschmutzung nach dem Aufbringen gerinnen gelassen und/oder getrocknet wird.

14. Verfahren nach Anspruch 12 oder 13, dadurch gekennzeichnet, daß der Nachweis von Resten der Testanschmutzung durch eine chemische Farbreaktion und anschließende visuelle Beurteilung erfolgt.

15. Verfahren nach Anspruch 14, dadurch gekennzeichnet, daß als chemische Farbreaktion die Biuret-Reaktion verwendet wird.

16. Kit zur Durchführung des Verfahrens nach einem der Ansprüche 12 bis 15, gekennzeichnet durch folgende Bestandteile:
- einem Prüfkörper, der mit einer synthetischen Testanschmutzung, die Fibrin und/oder eine Fibrinvorstufe in Verbindung mit einem Umwandlungsinitiator enthält, versehen ist,
- Nachweisreagenzien zum Nachweis von Resten der Testanschmutzung.

17. Kit nach Anspruch 16, dadurch gekennzeichnet, daß die Nachweisreagenzien enthalten:
- eine alkalische wäßrige Lösung,
- eine wäßrige Lösung von Cu²⁺.

18. Kit nach Anspruch 17, dadurch gekennzeichnet, daß die alkalische wäßrige Lösung zusätzlich einen Komplexbildner für Cu²⁺-Ionen enthält.

## Claims

1. Synthetic test stain, characterized in that it contains:
a) fibrin and/or a fibrin precursor in combination with a conversion initiator,
b) haemoglobin.

2. Synthetic test stain according to Claim 1, characterized in that the fibrin precursor is fibrinogen.

3. Synthetic test stain according to Claim 1 or 2, characterized in that it additionally contains blood plasma proteins.

4. Synthetic test stain according to Claim 3, characterized in that it contains albumin.

5. Use of a fibrin glue for the preparation of a synthetic test stain.

6. Kit for the preparation of a synthetic test stain, characterized by the following components:
a) a fibrin precursor,
b) a conversion initiator for the fibrin precursor,
at least one of these components additionally containing haemoglobin.

7. Kit according to Claim 6, characterized in that component a) contains fibrinogen.

8. Kit according to Claim 6 or 7, characterized in that component b) contains thrombin.

9. Kit according to one of Claims 6 to 8, characterized in that component a) additionally contains blood plasma proteins.

10. Kit according to Claim 9, characterized in that component a) contains albumin.

11. Kit according to one of Claims 6 to 10, characterized in that the components of the kit are present as a solution in isotonic saline.

12. Method of checking the efficiency of a cleaning process, characterized by the following steps:
a) applying a synthetic test stain, which contains fibrin and/or a fibrin precursor in combination with a conversion initiator, to a test sample,
b) subjecting the test sample to the cleaning process to be tested,
c) detecting residues of the test stain on the test sample.

13. Method according to Claim 12, characterized in that the test stain is left to coagulate and/or dried after application.

14. Method according to Claim 12 or 13, characterized in that residues of the test stain are detected by means of a chemical colour reaction and subsequent visual assessment.

15. Method according to Claim 14, characterized in that the chemical colour reaction used is the biuret reaction.

16. Kit for carrying out the method according to one of Claims 12 to 15, characterized by the following parts:
- a test sample provided with a synthetic test stain, which contains fibrin and/or a fibrin precursor in combination with a conversion initiator,
- analytical reagents for detecting residues of the test stain.

17. Kit according to Claim 16, characterized in that the analytical reagents contain:
- an alkaline aqueous solution,
- an aqueous solution of Cu²⁺.

18. Kit according to Claim 17, characterized in that the alkaline aqueous solution additionally contains a complexing agent for Cu²⁺ ions

## Revendications

1. Souillure synthétique pour test, caractérisée en ce qu'elle contient
a) de la fibrine et/ou un précurseur de la fibrine associé à un initiateur de conversion
b) de l'hémoglobine.

2. Souillure synthétique pour test selon la revendication 1, caractérisée en ce que le précurseur de la fibrine est du fibrinogène.

3. Souillure synthétique pour test selon les revendications 1 ou 2, caractérisée en ce qu'elle contient des protéines supplémentaires du plasma sanguin.

4. Souillure synthétique pour test selon la revendication 3, caractérisée en ce qu'elle contient de l'albumine.

5. Utilisation d'une colle de fibrine pour la préparation d'une souillure synthétique pour test.

6. Kit pour la préparation d'une souillure synthétique pour test, caractérisé par les composants suivants :
a) un précurseur de la fibrine,
b) un initiateur de conversion pour le précurseur de la fibrine,
au moins un de ces composants contenant en outre de l'hémoglobine.

7. Kit selon la revendication 6, caractérisé en ce que le composant a) contient du fibrinogène.

8. Kit selon les revendications 6 ou 7, caractérisé en ce que le composant b) contient de la thrombine.

9. Kit selon l'une quelconque des revendications 6 à 8, caractérisé en ce que le composant a) contient en outre des protéines du plasma sanguin.

10. Kit selon la revendication 9, caractérisé en ce que le composant a) contient de l'albumine.

11. Kit selon l'une quelconque des revendications 6 à 10, caractérisé en ce que les composants du kit se trouvent sous forme dissoute dans une solution isotonique de chlorure de sodium.

12. Procédé pour tester l'efficacité d'un procédé de nettoyage, caractérisé par les étapes suivantes :
a) application d'une souillure synthétique pour test, qui contient de la fibrine et/ou un précurseur de la fibrine, associé à un initiateur de conversion, sur un échantillon d'essai,
b) soumission de l'échantillon d'essai au procédé de nettoyage à tester,
c) détection des restes de la souillure pour test sur l'échantillon d'essai.

13. Procédé selon la revendication 12, caractérisé en ce qu'on laisse se coaguler et/ou on sèche la souillure pour test après l'application.

14. Procédé selon les revendications 12 ou 13, caractérisé en ce que la détection de restes de la souillure pour test est réalisée par une réaction de coloration chimique et une évaluation visuelle consécutive.

15. Procédé selon la revendication 14, caractérisé en ce qu'on utilise comme réaction de coloration chimique la réaction du biuret.

16. Kit pour réaliser le procédé selon l'une quelconque des revendications 12 à 15, caractérisé par les constituants suivants :
- un échantillon d'essai, qui est pourvu d'une souillure synthétique pour test, qui contient de la fibrine et/ou un précurseur de la fibrine associé à un initiateur de conversion,
- des réactifs de détection pour détecter des restes de la souillure pour test.

17. Kit selon la revendication 16, caractérisé en ce que les réactifs de détection contiennent :
- une solution aqueuse alcaline,
- une solution aqueuse de Cu²⁺.

18. Kit selon la revendication 17, caractérisé en ce que la solution aqueuse alcaline contient en outre un complexant pour les ions Cu²⁺.
